# EUROPEAN PATENT APPLICATION

(11) **EP 2 447 241 A1**
(43) Date of publication of application: **02.05.2012**
(21) Application number: 10013766.0
(22) Date of filing: 19.10.2010
(51) Int. Cl.: C07C 51/14, C07C 67/08, C07C 53/128, C07C 69/24

(54) **Glycidyl esters of alpha, alpha branched neononanoic acids, synthesis and uses**

(71) Applicant: Momentive Specialty Chemicals Research Belgium, 1348 Ottignies Louvain-la-Neuve (BE)
(72) Inventor: Le Fevere De Ten Hove, Cédric, 1348 Ottignies Louvain-la-Neuve (BE); Heymans, Denis, 1348 Ottignies Louvain-la-Neuve (BE); Steinbrecher, Christophe, 1348 Ottignies Louvain-la-Neuve (BE); Kotlewska, Aleksandra, 3196 KK Vondelingenplaat RT (NL); Van't Sand, Robert, 3196 KK Vondelingenplaat RT (NL)

(57) **Abstract**

The invention relates to a manufacturing process for the preparation of α,α-branched alkane carboxylic acids providing glycidyl esters with an improved softness or hardness of the coatings derived thereof.

In which the mixture of neononanoic acid providing a high hardness is a mixture where the sum of the concentration of the blocked and of the highly branched isomers is at least 55%, preferably above 65%. In which a mixture of neononanoic acids providing soft polymers is a mixture where the concentration of blocked and highly branched isomers is maximum 55% preferably below 40%.

## Description

The present invention relates to a manufacturing process for the preparation of α,α-branched alkane carboxylic acids providing glycidyl esters with an improved softness or hardness of the coatings derived thereof.

More in particular the invention relates to the preparation of aliphatic tertiary saturated carboxylic acids or α,α-branched alkane carboxylic acids, which contain 9 or 13 carbon atoms and which provide glycidyl esters with a branching level of the alkyl groups depending on the olefin feedstock used, and which is defined as below.

It is generally known from e.g. US 2,831,877, US 2,876,241, US 3,053,869, US 2,967,873 and US 3,061,621 that mixtures of α,α-branched alkane carboxylic acids can be produced, starting from mono-olefins, carbon monoxide and water, in the presence of a strong acid.

From e.g. H van Hoorn and G C Vegter, Dynamic modulus measurements as a tool in the development of paint base materials FATIPEC, Euro Continental Congress 9, p. 51-60 (1968); Rheol. Acta 10, p. 208-212 (1971); H van Hoorn, the influence of side group structure on the glass transition temperature of isomeric vinyl ester polymers, the relation between the final coating film properties and the isomer distribution in starting branched carboxylic acids, was known.

In such mixtures of α,α-branched alkane carboxylic acids, significant proportions of blocking ß-methyl-branched carboxylic acid isomers were found, the properties of which have been found to antagonize the attractive properties of other α,α-branched saturated carboxylic acid constituents of said mixtures, when applied in the form of vinyl esters in the coating industry, requiring more and more so-called softer acid derivatives.

More in particular the conventionally produced α,α-branched carboxylic acid mixtures had been found to cause a too high hardness of the final coating of films, which was disadvantageous and therefore undesired for certain applications, due to the presence of significant proportions of blocking β-alkyl-branched isomers.

One of the more recent remedies has been disclosed in EP 1033360A1. The problem of providing better softening derivatives of α,α-branched acids, manufactured from alkenes, carbon monoxide and water and an acid catalyst was solved therein by a process, which actually comprised:
(a) oligomerisation of butene;
(b) separation of butene dimers and/or trimers from the oligomerisate;
(c) conversion of the butene dimers and/or trimers into carboxylic acids;
(d) conversion of the carboxylic acids into the corresponding vinyl esters showing attractive softening properties when mixed into other polymers or if used as comonomers in coatings.

An object of the above process are branched carboxylic acids, which are prepared from butene dimers obtainable by the OCTOL oligomerisation process and containing not more than 35 wt% of multi-branched olefins, such as dimethylhexene, and preferably at most 25 wt%. Moreover said carboxylic acids, having a significantly increased content of 2,2-dimethyl heptanoic acid and 2-ethyl,2-methyl hexanoic acid, and a decreased content of 2,3-dimethyl-2-ethyl pentanoic acid, could provide the presently required attractive properties of the corresponding vinyl esters, such as a Tg of -3°C at the lowest for the homopolymer of said C₉ vinyl ester.

Another object of the disclosed process in EP 1 033 360 are branched C₁₃ carboxylic acids, which are prepared from butene trimers obtainable by the OCTOL oligomerisation process. Moreover said carboxylic acids, could provide the required attractive properties of the corresponding vinyl esters, such as a Tg of -13°C at the lowest for the homopolymer of said C₁₃ vinyl ester.

The entire prior art is interested to provide soft monomers to be used as vinyl ester in latex formulations for example and to act as plasticizer.

An object of the present invention is to provide α,α-branched alkane carboxylic acids glycidyl ester derivatives in order to obtain attractive properties of coatings derived therefrom.

As a result of extensive research and experimentation a process giving the branched carboxylic acids aimed at, it has surprisingly been found, that the branching level of the glycidyl ester has a strong influence on the coating properties.

Accordingly, the invention relates to a manufacturing process for the preparation of α,α-branched alkane carboxylic acids, by reacting a mono-olefin or a precursor thereof, with carbon monoxide in the presence of a catalyst and water characterized in that the starting olefin is ethylene or ethylene oligomers, or butene or butene derivatives or butene precursors ( such as alcohol), the most preferred are butene or butene oligomers. The industrial sources for butene are Raffinate I or Raffinate II or Raffinate III.

Raf I or any other mixture of alkane-alkene with a isobutene content of at least 50% weight on total alkene, are fractions used to produce a highly branched acid derivatives after dimerisation or trimerisation, carboxylation and subsequently glycidation, the mixture of neo-acid (C9 or C13 acids) derivatives obtained by such a process and providing a mixture where the sum of the concentration of the blocked and of the highly branched isomers is at least 55%, preferably above 65%.

If the olefin feed is based on Raf. II or Raf III or any mixture rich in n-butene isomers on the total olefins, the subsequently mixture of neo-acid (C9 or C13 acids) derivatives will provide a mixture where the concentration of blocked and highly branched isomers is maximum 55% preferably below 40%.

We have discovered that well chosen blend of isomers of the glycidyl ester of, for example, neononanoic acids give different and unexpected performance in combination with some particular polymers such as polyester polyols. The isomers are described in Table 1 and illustrate in Figure 1.

From the prior art it is known that polyester resins based on the commercially available Cardura E10 often result in coatings with a low hardness and a poor drying speed. This low drying speed results in work time lost when the coating is applied e.g. on cars and other vehicles. One alternative to this drawback was suggested in the literature by using the pivalic glycidyl ester (EP 0 996 657), however this low molecular derivative is volatile.

There is also a need for glycidyl esters giving a lower viscosity to the derived polyesters or ether resins and epoxy systems but that are free of any safety risks.

We have found that the performance of the glycidyl ester derived from the branched acid is depending on the branching level of the alkyl groups R¹, R² and R³, for example the neononanoic acid has 3, 4 or 5 methyl groups. Highly branched isomers are defined as isomers of neo-acids having at least 5 methyl groups.

Mixture of neononanoic acid providing a high hardness is a mixture where the sum of the concentration of the blocked and of the highly branched isomers is at least 55%, preferably above 65%.

Mixture of neononanoic acids providing soft polymers is a mixture where the concentration of blocked and highly branched isomers is maximum 55% preferably below 40%.

The desired isomer distribution can be obtained by the selection of the correct starting olefin or precursors thereof, and also to a lesser extend by adjusting the Kock reaction conditions. The dimerisation or trimerisation of the olefin and/or the precussor thereof is done according the method of EP1033360 and the subsequently carbonylation will provide the desired branched acid, which can be glycidated according to PCT/EP2010/003334 or the US6433217.

The glycidyl esters so obtained can be used as reactive diluent for epoxy based formulations such as examplified in the technical brochure of Hexion(Product Bulletin: Cardura N10 The Unique Reactive Diluent). Other uses of the glycidyl ester are the combinations with polyester polyols, or acrylic polyols, or polyether polyols. The combination with polyester polyols such as the one used in the car industry coating leads to a fast drying coating system with attractive coating properties.

The invention is further illustrated by the following examples, however without restricting its scope to these embodiments.

### Methods used

The isomer distribution of neo-acid can be determined using by gas chromatography, using a flame ionization detector (FID). 0.5 ml sample is diluted in analytical grade dichloromethane and n-octanol may be used as internal standard. The conditions presented below result in the approximate retention times given in table 1. In that case n-Octanol has a retention time of approximately 8.21 minute.

The GC method has the following settings:
Column: CP Wax 58 CB (FFAP), 50 m x 0.25 mm, df = 0.2 µm
Oven program : 150°C (1.5 min) - 3.5°C/min - 250°C (5 min) = 35 min
Carrier gas: Helium
Flow : 2.0 mL/min constant
Split flow : 150 mL/min
Split ratio: 1:75
Injector temp : 250°C
Detector temp : 325°C
Injection volume: 1 µL

CP Wax 58 CB is a Gas chromatography column available from Agilent Technologies.

The isomers of neononanoic acid as illustrative example have the structure (R¹ R² R³) C COOH where the three R groups are linear or branched alkyl groups having together a total of 7 carbon atoms. The structures and the retention time, using the above method, of all theoretical possible neononanoic isomers are drawn in Figure 1 and listed in table 1.

**Table 1: Structure of all possible neononanoic isomers**

| | **R1** | **R2** | **R3** | **Methyl groups** | **Blocking** | **Retention time [Minutes]** |
|---|---|---|---|---|---|---|
| V901 | Methyl | Methyl | n-pentyl | 3 | No | 8.90 |
| V902 | Methyl | Methyl | 2-pentyl | 4 | Yes | 9.18 |
| V903 | Methyl | Methyl | 2-methyl butyl | 4 | No | 8.6 |
| V904 | Methyl | Methyl | 3-methyl butyl 1,1-dimethyl | 4 | No | 8.08 |
| V905 | Methyl | Methyl | propyl 1,2-dimethy | 5 | Yes | 10.21 |
| V906 | Methyl | Methyl | propyl 2,2-dimethyl | 5 | Yes | 9.57 |
| V907 | Methyl | Methyl | propyl | 5 | No | 8.26 |
| V908 | Methyl | Methyl | 3-pentyl | 4 | Yes | 9.45 |
| V909 V910 | Methyl | Ethyl | n-butyl | 3 | No | 9.28 |
| K1 | Methyl | Ethyl | s-butyl | 4 | Yes | 9.74 |
| V910 | | | | | | |
| K2 | Methyl | Ethyl | s-butyl | 4 | Yes | 9.84 |
| V911 | Methyl | Ethyl | i-butyl | 4 | No | 8.71 |
| V912 | Methyl | Ethyl | t-butyl | 5 | Yes | 9.64 |
| V913 | Methyl | n-propyl | n-propyl | 3 | No | 8.96 |
| V914 | Methyl | n-propyl | i-propyl | 4 | Yes | 9.30 |
| V915 | Methyl | i-propyl | i-propyl | 5 | Yes | 9.74 |
| V916 | Ethyl | Ethyl | n-propyl | 3 | No | 9.44 |
| V917 | Ethyl | Ethyl | i-propyl | 4 | Yes | 10.00 |

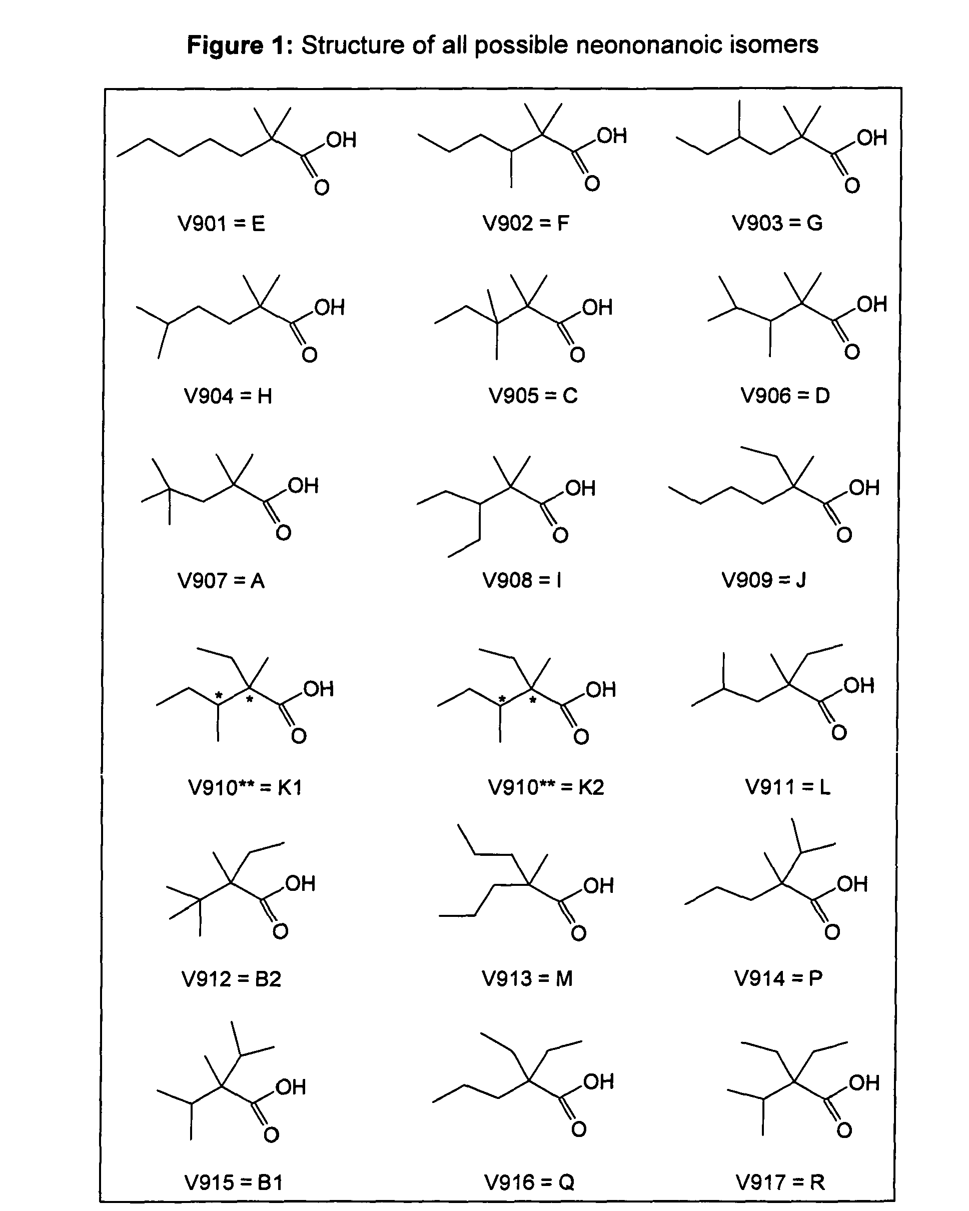

### Blocking isomers

Whereas the carbon atom in alpha position of the carboxylic acid is always a tertiary carbon atom, the carbon atom(s) in **β** position can either be primary, secondary or tertiary. Neononanoic acids (V9) with a secondary or a tertiary carbon atoms in the **β** position are defined as blocking isomers (Figures 2 and 3).

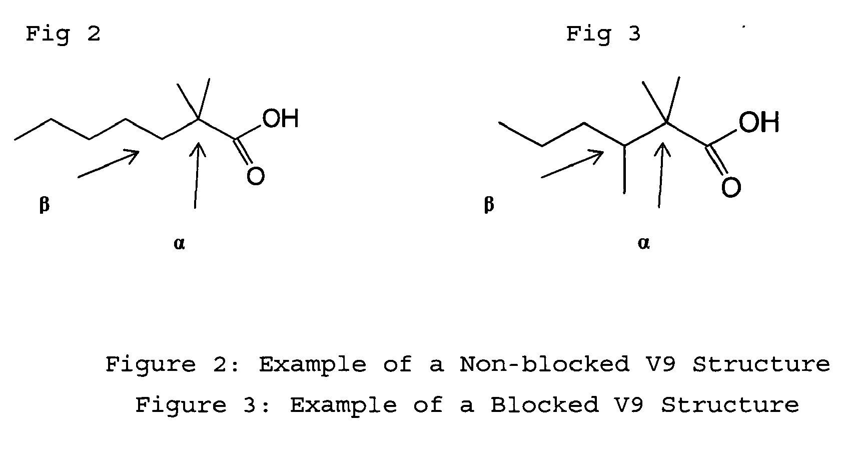

### Methods for the characterization of the resins

The molecular weights of the resins are measured with gel permeation chromatography (Perkin Elmer/ Water) in THF solution using polystyrene standards. Viscosity of the resins are measured with Brookfield viscometer (LVDV-I) at indicated temperature. Solids content are calculated with a function (Ww-Wd) / Ww x 100%. Here Ww is the weight of a wet sample, Wd is the weight of the sample after dried in an oven at a temperature 110 °C for 1 hour.

### Methods for the Characterization of the Clear Coats

### Pot-life

Pot-life is determined by observing the elapsed time for doubling of the initial viscosity at room temperature, usually 24.0 ± 0.5 °C. The initial viscosity of the clear coat is defined at 51.3 - 58.5 mPa.s for Part 1 and 93-108 mPa.s for Part 3 measured with Brookfield viscometer.

### Application of clearcoat

Q-panels are used as substrates. Then the panels are cleaned by a fast evaporating solvent methyl ethyl ketone or acetone. The clearcoat is applied with a barcoater.

### Dust free time

The dust free time (DFT) of clear coat is evaluated by vertically dropping a cotton wool ball on a flat substrate from a defined distance. When the cotton ball contacts with the substrate, the substrate is immediately turned over. The dust free time is defined as the time interval at which the cotton wool ball no longer adhered to the substrate.

### Hardness development

Hardness development is followed using pendulum hardness tester with Koenig method. **Chemicals used:**

### Curing agents

**HDI:** 1,6-hexamethylene diisocyanate trimer, Desmodur N3390 BA from Bayer Material Science or Tolonate HDT LV2 from Perstorp
**Leveling agent**: 'BYK 10 wt%' which is BYK-331 diluted at 10% in butyl acetate
**Catlyst:** 'DBTDL 1 wt%' which is Dibutyl Tin Dilaurate diluted at 1wt% in butyl acetate
**Thinner: A**: is a mixture of Xylene 50wt%, Toluene 30wt%, ShellsolA 10wt%,2-Ethoxyethylacetate 10wt%.
   **B**: is butyl acetate
**Monopentaerythritol:** available from Sigma - Aldrich **Methylhexahydrophtalic anhydride:** available from Sigma - Aldrich
**acrylic acid :** available from Sigma - Aldrich
**hydroxyethyl methacrylate:** available from Sigma - Aldrich
**styrene:** available from Sigma - Aldrich
**2-ethylhexyl acrylate :** available from Sigma - Aldrich
**methyl methacrylate :** available from Sigma - Aldrich
**tert-Butyl peroxy-3,5,5-trimethylhexanoate:** available from Akzo Nobel
**Cardura™ E10**: available from Hexion Specialty Chemicals
**GE9S:** glycidyl ester of C9 neo-acids obtained by dimerisation of n-butene, or Raf. II or Raf. III (leading to a mixture where the concentration of blocked and highly branched isomers is maximum 55% preferably below 40%) in presence of CO, a catalyst, water and subsequently reacted with epichlorohydrin
**GE9H**: glycidyl ester of C9 neo-acids obtained by dimerisation of iso-butene, or Raf. I (leading to a mixture where the concentration of the blocked and of the highly branched isomers is at least 55%, preferably above 65%) in presence of CO, a catalyst, water and subsequently reacted with epichlorohydrin
**GE5:** glycidyl ester of pivalic acid obtained by reaction of the acid with epichlorhydrin.

### EXAMPLES

### Example 1

### Monopentaerythritol / Methylhexahydrophtalic anhydride / GE9S (1/3/3 molar ratio) = CE-GE9S

80.4 g amount of butylacetate, 68.3 g of monopentaerythritol, 258.2 g of methylhexahydrophthalic anhydride are loaded in a glass reactor and heated to reflux until complete dissolution. Afterwards, the temperature is decreased down to 120°C and 333.0 g of GE9S are added over about one hour. The cooking is pursued at 120°C for the time needed to decrease epoxy group content and acid value down to an acid value below 15 mg KOH/g. Then, further 82.4 g of butylacetate are added. Test results are indicated in table 2.

### Example 2

### Monopentaerythritol / Methylhexahydrophtalic anhydride / GE9H (1/3/3 molar ratio) = CE-GE9H

80.4 g amount of butylacetate, 68.3 g of monopentaerythritol, 258.2 g of methylhexahydrophthalic anhydride are loaded in a glass reactor and heated to reflux until complete dissolution. Afterwards, the temperature is decreased down to 120°C and 337.1 g of GE9H are added over about one hour. The cooking is pursued at 120°C for the time needed to decrease epoxy group content and acid value down to an acid value below 15 mg KOH/g. Then, further 83.4 g of butylacetate are added.

Comparative example according to EP 0996657 Monopentaerythritol / Methylhexahydrophtalic anhydride / GE5 (1/3/3 molar ratio) CE-GE5

71.3 g amount of butylacetate, 60.5 g of monopentaerythritol, 228.90 g of methylhexahydrophthalic anhydride are loaded in a glass reactor and heated to reflux until complete dissolution. Afterwards, the temperature is decreased down to 120°C and 214.3 g of GE5 are added over about one hour. The cooking is pursued at 120°C for the time needed to decrease epoxy group content and acid value down to an acid value below 15 mg KOH/g. Then, further 52.1 g of butylacetate are added.

**Table 2: Polyesters characterization**

| **Polyester resin** | **SC(%)** | **Mw (Da)** | **Mn (Da)** | **Mw/Mn (PDI)** | **Viscosity (cP)** |
|---|---|---|---|---|---|
| CE-GE9S | 78.6 | 974 | 919 | 1.06 | 2450 |
| CE-GE9H | 80.0 | 921 | 877 | 1.05 | 6220 |
| CE-GE5 | 79.3 | 914 | 886 | 1.03 | 5080 |

| | | | | | |
|---|---|---|---|---|---|
| SC : solids content | | | | | |

### Example 3

### Acrylic resin synthesis

### Cardura™ E10 based acrylic polyol resin: Acryl-CE(10)

45.0 g amount of CE10 (Cardura™ E10-glycidyl ester of Versatic acid) and 99.1 g of n-butyl acetate are loaded in a glass reactor and heated up to 120°C. Then, a mixture of monomers (17.9g acrylic acid, 83.9g hydroxyethyl methacrylate, 90.0g styrene, 135.9 g 2-ethylhexyl acrylate, 84.6g methyl methacrylate) and initiator (4.5g tert-Butyl peroxy-3,5,5-trimethylhexanoate) is fed into the reactor at a constant rate in 5 hours. Then post cooking started: a mixture of 4.5g tert-Butyl peroxy-3,5,5-trimethylhexanoate and 13.6g n-butyl acetate is fed into the reactor at a constant rate in 0.5 hour, then temperature maintained at 120°C for a further 0.5 hour. Finally, 353.7g of n-butyl acetate is added under stirring to achieve a polyol resin with the target solids content. Test results are indicated in table 3.

**Table 3: Acryl-CE(10) characterization**

| **Acryl-** | **SC (%) - measured** | **Mw (Da)** | **Mn. (Da)** | **Mw/Mn (PDI).** |
|---|---|---|---|---|
| CE (10) | 49.0 | 44428 | 15552 | 2.86 |

### Formulation of the Clear Coats

A clear coat is formulated with one of the polyesters (CE-GEX), optionally the Acryl-CE(10) resin, the curing agent, the thinner, the levelling agent (BYK-331) and the catalyst (dibutyltin dilaurate, DBTDL) according to the amounts indicated in tables 4, 5 and 6.

Three types of formulations have been prepared:
■ Blend Acryl-CE(10) blend with CE-GEx polyester with Desmodur as hardener (**Part 1**)
■ CE-GEx polyester alone with Tolonate HDT LV2 as hardener (**Part 2**)
■ CE-GEx polyester alone with Tolonate HDT LV2 as hardener (0.09wt% DBTDL) (**Part 3**)

### Part 1: CE-GEx polyesters blend with Acryl-CE(10) Formulation

**Table 4: Clear coats, formulations (Part 1 - CE-GEx polyesters blend with Acryl-CE(10)**

| **CE-GEx** | **Binder 1 (g)** | **Binder 2 (g)** | **HDI (g)** | **BYK 10 wt% (g)** | **DBTDL 1wt% (g)** | **Thinner A (g)** |
|---|---|---|---|---|---|---|
| **GE9S** | 49.1 | 78.5 | 23.05 | 0.5100 | 1.1200 | 92.37 |
| **GE9H** | 49.0 | 80.2 | 23.10 | 0.5100 | 1.1200 | 86.33 |
| **GE5** | 49.0 | 79.2 | 23.30 | 0.5100 | 1.1200 | 89.33 |

| | | | | | | |
|---|---|---|---|---|---|---|
| Binder 1: Acryl-CE(10) Binder 2: CE-GEx polyesters | | | | | | |

### Part 2 - CE-GEx polyesters alone, no Acryl-CE(10) Formulation

**Table 5: Clear coats, formulations (Part 2 - CE-GEx polyesters alone)**

| **CB=GBx:** | **Binder 2 (g)** | **HDI (g)** | **BYK 10wt%** | DBTDL **1%wt (g)** | **Thinner B (g)** |
|---|---|---|---|---|---|
| **GE9S** | 80.0 | 36.56 | 0.72 | 3.15 | 89.75 |
| **GE9H** | 80.4 | 37.27 | 0.73 | 3.20 | 87.83 |
| **GE5** | 79.9 | 43.18 | 0.76 | 3.36 | 94.82 |

### Part 3 - CE-GEx polyesters alone, no Acryl-CE(10) Formulation (0.09wt% DBTDL)

**Table 6: Clear coats, formulations (Part 3 - CE-GEx polyesters alone)**

| **CE-GEx** | **Binder 2 (g)** | **HDI (g)** | **BYK 10 wt% (g)** | **DBTDL (g)** | **Thinner B (g)** |
|---|---|---|---|---|---|
| GE9H | 60.0 | 28.10 | 0.54 | 7.18 | 15.40 |
| **GE5** | 59.8 | 32.54 | 0.57 | 7.57 | 17.79 |

### Characterization of the Clear Coats

The clearcoat formulations are barcoat applied on degreased Q-panel. The panels are dried at room temperature, optionally with a preliminary stoving at 60°C for 30 min.

### Part 1 - CE-GEx polyesters blend with Acryl-CE(10) / Room temperature curing

**Table 7: Clear coats, performances (Part 1 - CE-GEx polyesters blend with Acryl-CE(10)**

| **CE-GEx** | **SC** (%) | **Potlife** (min) | **Drying conditions** | DFT (min) | **Koenig Hardness (s)** | | |
|---|---|---|---|---|---|---|---|
| | | | | *Cotton Balls* | **6h** | **24h** | **7d** |
| GE9S | 44.9 | 160.8 | RT | 28 | 4.4 | 16.9 | 38.9 |
| GE9H | 45.6 | 174.9 | RT | 14 | 5.4 | 25.3 | 69.5 |
| GE5 | 44.1 | 195.7 | RT | 20 | 4.0 | 18.2 | 62.1 |

| | | | | | | | |
|---|---|---|---|---|---|---|---|
| SC: solids content, RT: room temperature | | | | | | | |

### Part 2 - CE-GEx polyesters alone, no Acryl-CE(10) / Room temperature curing and room temperature drying after stoving

**Table 8: Clear coats, performances (Part 2 - CE-GEx polyesters alone, no Acryl-CE(10)**

| **CE-GEx** | **SC (%)** | **Drying conditions** | **DFT** (min) | **Koenig Hardness**(s) | | |
|---|---|---|---|---|---|---|
| | | | *Cotton Balls* | **6h** | **24h**. | **7d** |
| GE9S | 48.4 | RT | 223 | 3 | 17 | 159 |
| GE9H | 49.2 | RT | 91 | 3 | 36 | 212 |
| GE5 | 49.5 | RT | 114 | 1 | 29 | 216 |
| | | | | | | |
| GE9S | 48.4 | Stoving 30 min/60°C | Dust free out of oven | 4 | 44 | 174 |
| GE9H | 49.2 | Stoving 30 min/60°C | Dust free out of oven | 10 | 55 | 211 |
| GE5 | 49.5 | Stoving 30 min/60°C | Dust free out of oven | 6 | 49 | 21 £ |

### Part 3 - CE-GEx polyesters alone, no Acryl-CE(10) / Room temperature curing and room temperature drying after stoving (0.09wt% DBTDL)

**Table 9: Clear coats, performances (Part 3- CE-GEx polyesters alone, no Acryl-CE(10))**

| **CE-GEx** | **SC** (%) | **Potlife** (min) | **Drying conditions** | **DFT** (min) | **Koenig Hardness** (s) | | |
|---|---|---|---|---|---|---|---|
| | | | | *Cotton Balls* | **6h** | **24h** | **7d** |
| GE9H | 69.8 | 42.4 | RT | 47 | 3 | 66 | 193 |
| GE5 | 68.5 | 61.3 | RT | 73 | 3 | 55 | 191 |
| | | | | | | | |
| GE9H | 69.8 | 42.4 | Stoving 30 min/60°C | Dust free out of oven | 29 | 102 | 210 |
| GE5 | 68.5 | 61.3 | Stoving 30 min/60°C | Dust free out of oven | 12 | 69 | 205 |

### Observations

### Part 1

The potlife is about the same, the hardness development is from the highest to the lowest in the following order GE9H, GE5 and GE9S, the same ranking order for the dust free time.

### Part 2

The 24h hardness order GE9H, GE5 and GE9S and the dust free time at room temperature is the best for GE9H.

### Part 3

The hardness development is the best for GE9H at room temperature and heat cure, the dust free time at room temperature is quicker for GE9H than for GE5; and with a volatile organic content of 300 g/1.

## Claims

1. A process for synthesis of glycidyl ester from butene oligomers, comprising:
(a) oligomerizing butenes or precursors of butene in presence of a catalyst,
(b) converting butene oligomers to carboxylic acids which are longer by one carbon atom, and
(c) converting the carboxylic acids to the corresponding glycidyl esters.

2. The process of claim 1 **characterized in that** the olefin has a weight fraction of isobutene of at least 50 weight% on total alkene fraction of the mixture feed, or that the olefin has a weight fraction of n-butene isomers of at least 50 weight% on total alkene fraction of the mixture feed.

3. The process of claims 1 and 2 **characterized in that** the olefin has a weight fraction of isobutene of at least 50% and that the subsequently mixture of neo-acid (C9 or C13 acids) derivative obtained by such a process is providing a mixture where the sum of the concentration of the blocked and of the highly branched isomers is at least 55%, preferably above 65%.

4. The process of claims 1 and 2 **characterized in that** the olefin has a weight fraction of n-butene of at least 50% and the subsequently mixture of neo-acid (C9 or C13 acids) derivatives will provide a mixture where the concentration of blocked and highly branched isomers is maximum 55% preferably below 40%.

5. A binder composition comprising the glycidyl esters obtained according to claims 1 to 4 reacted in resins such as an hydroxyl functional polyester, or an hydroxyl functional polyether, or an hydroxyl functional acrylic resins or a mixture thereof.

6. The use of the glycidyl esters obtained according to claims 1 to 4 in blend with epoxy resins as reactive diluent.

7. The binder of claim 5 in which the hydroxyl polyester is **characterized by** a calculated hydroxyl value above 100 mgKOH/g and an average molecular weight (MW) lower as 5 000 DA measured according the above method (polystyrene standard).

8. The binder of claim 5 in which the hydroxyl polyether is **characterized by** a calculated hydroxyl value above 120 mgKOH/g and an average molecular weight (MW) lower as 4 500 DA measured according the above method (polystyrene standard).

9. The binder of claim 5 in which the hydroxyl functional acrylic resins is **characterized by** a calculated hydroxyl value between 50 and 180 mgKOH/g and an average molecular weight (MW) between 2 500 and 50 000 DA measured according the above method (polystyrene standard).

10. The binder of claims 7 to 9 in which the subsequently mixture of neo-acid (C9 or C13 acids) glycidyl derivative obtained by the process of claim 1 has an isomer composition wherein the sum of the concentration of the blocked and of the highly branched isomers is at least 55%, preferably above 65%.

11. The binder of claims 7 to 9 in which the subsequently mixture of neo-acid (C9 or C13 acids) glycidyl derivative obtained by the process of claim 1 has an isomer composition wherein the sum of the concentration of concentration of blocked and highly branched isomers is maximum 55% preferably below 40%.
